# EUROPEAN PATENT SPECIFICATION

(11) **EP 1 814 513 B1**
(45) Date of publication and mention of the grant of the patent: **15.07.2009**
(21) Application number: 05807450.1
(22) Date of filing: 21.11.2005
(51) Int. Cl.: A61K 8/25, A61Q 1/00

(54) **PULVERULENT COSMETIC FORMULATION HAVING A HIGH WATER CONTENT**
PULVERFÖRMIGE KOSMETISCHE FORMULIERUNG MIT HOHEM WASSERGEHALT
FORMULATION COSMETIQUE PULVERULENTE A HAUTE TENEUR EN EAU

(30) Priority: 25.11.2004 DE 102004056862
(43) Date of publication of application: 08.08.2007
(73) Proprietor: Evonik Degussa GmbH, 45128 Essen (DE)
(72) Inventor: HASENZAHL, Steffen, Morris Plains, NJ 07950 (US); READER, Stephanie, 63452 Hanau (DE); GRAY, Ann, 63452 Hanau (DE)
(86) International application number: PCT/EP2005/012419
(87) International publication number: WO 2006/056377

(56) References cited:
- EP-A- 0 855 177
- EP-A- 1 206 928
- WO-A-20/04028250
- JP-A- 2000 309 505
- US-A1- 2001 047 047
- S. HASENZAHL ET AL.: "Fumed silica for personal care and cosmetics" SÖFW-JOURNAL, no. 8, 2003, pages 1-8, XP002289365 cited in the application
- Factsheet "Aeroperl 300/30", Evonik Ind., Feb.2007.

## Description

The invention relates to a pulverulent cosmetic formulation which has a water content of at least 50 wt.% and comprises a hydrophobized silicon dioxide powder.

It is known to prepare solid cosmetic formulations, such as powders, having a high water content. The basis of this is the long-known fact that, in the presence of hydrophobized silicon dioxide powder, water is divided into fine droplets and enveloped, and joining together of the drops of water is thereby prevented. A pulverulent substance, so-called "dry water" (publication series Fine Particles No. 11, Degussa AG, Düsseldorf) is formed in this manner.

The use of aqueous solutions of pharmaceutically or cosmetically relevant substances for the preparation of "dry water" is described for the first time in DE 1467023. In this, a hydrophobized silicon dioxide powder having a BET surface area of 150 m²/g and a carbon content of from 1.0 to 1.5 wt.%, which is prepared by reaction with dimethyldichlorosilane, is used.

US 3393155 furthermore teaches the use of water-miscible liquid components, acids and salts for the preparation as well as the possible use of such formulations in cosmetics based on "dry water". Here also, a hydrophobized silicon dioxide powder having a carbon content of approx. 1 wt.% and a BET surface area of approx. 150 m²/g is used.

EP 1 206 928 describes a water-containing powder formulation which comprises an aqueous gel core enveloped by hydrophobized silicon dioxide particles. AEROSIL® R974, AEROSIL® R972, AEROSIL® RX200, AEROSIL® RX300 and Cab-O-Sil® TS530 are mentioned as suitable products which are commercially available.

JP 2000-309505 discloses a cosmetic powder which comprises 2-20 wt.% of hydrophobized silicon dioxide powder having a BET surface area of greater than 60 m²/g, one or more oil-in-water emulsions and 0.001-10 wt.% of compounds which are not stable in the presence of water, for example a bleaching agent or an antiinflammatory agent. Purified water and a water-soluble polymer, such as gum arabic or carboxymethyl-starch, are preferred here. Avocado oil, camomile oil etc. are used as the oil phase.

EP 1 235 554 B1 describes a cosmetic or pharmaceutical powder-to-liquid composition of silicon dioxide particles preferably hydrophobized with alkylsilanes, water, a water-soluble polymer and less than 1 wt.% of oil.

JP 2002-265388 describes a substance which promotes transdermal absorption and is based on dry water, and which comprises a water-soluble medicinal active compound and a hydrophobized silicon dioxide powder having a surface area of more than 60 m²/g.

JP 2003-267826 describes a pulverulent cosmetic product with 0.1-20 wt.% of hydrophobized silicon dioxide powder having a BET surface area of more than 60 m²/g, 20-60 wt.% of a further pulverulent component and 40-80 wt.% of an aqueous component.

WO-A-200185138 discloses a system comprising a core of a water-containing liquid and a shell of hydrophobized solid particles.

EP-A-855177 discloses a powder for lightening skin which comprises 0.1-7 wt.% trimethylsiloxylated silicon dioxide powder having a surface area of at least 80 m²/g and a degree of hydrophobization of at least 50 %, 5-40 wt.% of a polyalcohol, 50-95 wt.% of water and 0.01 to 5 wt.% of a skin-lightening agent. AEROSIL® R812 and Cab-O-Sil® TS530 are mentioned as suitable commercial products.

Cosmetic formulations based on "dry water" are described in Seifen, Fette, Ole, Wachse (SÖFW), 8 (2003), pages 1-8. These are flowable, fine powders which liquefy when rubbed on the skin. AEROSIL® R812S is employed for this.

In many cases, water is separated out during storage of the pulverulent cosmetic formulations based on "dry water" of the prior art. Such formulations then cannot be commercialized, since they do not meet the minimum storage stability required of cosmetics, e.g. storage stability of three months at 45 ºC

Further disadvantages of the prior art described here are the high outlay on transportation and storage for the hydrophobized silicon dioxide powder, the high dust pollution during processing of the hydrophobized silicon dioxide powder and the long times for incorporation of the hydrophobized silicon dioxide powder.

The object of the invention was therefore to provide a pulverulent cosmetic formulation which reduces the disadvantages of the prior art.

The invention provides a pulverulent cosmetic formulation comprising at least one hydrophobized silicon dioxide powder, at least one cosmetically relevant constituent and at least 50 wt.% of water, based on the total amount of the formulation, in which the hydrophobized silicon dioxide powder has a tamped density of at least 70 g/l, determined in accordance with DIN EN ISO 787-11.

Further, said hydrophobized silicone dioxide powder has the characteristics disclosed in present claim 1.

On its addition to water a pulverulent product is formed and the tamped density of the hydrophobized silicon dioxide powder is greater than 70 g/l. The hydrophobized silicon dioxide powder is silanized.

In particular, the following substance is employed as silazane: 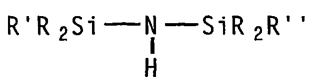 where R = R' = methyl, hexamethyldisilazane (for example DYNASYLAN^{®} HMDS).

The silanization can be carried out by spraying the silicon dioxide powder with the silanizing agent, which can optionally be dissolved in an organic solvent, such as, for example, ethanol, and then heat-treating the mixture at a temperature of from 105 to 400 ºC over a period of from 1 to 6 h.

The silicon dioxide powders employed for the hydrophobizing are of pyrogenic origin pyrogenic here includes those powders which are obtainable from suitable silicon compounds by flame oxidation or flame hydrolysis. As a rule, silicon tetrachloride is hydrolysed to silicon dioxide in a flame of hydrogen and oxygen.

The formulation according to the invention can preferably have a tamped density of from 70 g/l to 250 g/l, and particularly preferably of from 90 to 180 g/l.

Suitable commercially obtainable hydrophobized silicon dioxide powders can be Aerosil® R812 VV90 (90), Aerosil® R812S W90 (90), Aerosil® R8200 (140), all Degussa. Tamped density in g/l in parentheses.

Tamped densities in the range from at least 70 g/l can be obtained, for example, by mechanical after-treatment of the silicon dioxide powder before or after the hydrophobizing. This can be a compaction, a structure modification or a granulation.

Preferred compaction processes are described, for example, in DE-A-3238427 and DE-A-3741846. Hydrophobized silicon dioxide powders which are compacted by means of the vacuum rotary filter described in DE-A-3741846, which is equipped with a pressing belt, are particularly advantageous.

The BET surface area of the hydrophobized silicon dioxide powder is from 100 m²/g to 400 m²/g_{.}

The hydrophobized silicon dioxide powder in the formulation according to the invention has a methanol wettability of at least 40. In the determination of the methanol wettability, in each case 0.2 g (± 0.005 g) of hydrophobic silicon dioxide powder is weighed into transparent centrifuge tubes. 8.0 ml portions of a methanol/water mixture with 10, 20, 30, 40, 50, 60, 70 or 80 vol.% methanol are added to each sample weighed out. The closed tubes are shaken for 30 seconds and then centrifuged at 2,500 min⁻¹ for 5 minutes. The sediment volumes are read off, converted into per cent and plotted on a graph against the methanol content (vol.%). The point of inflection of the curve corresponds to the methanol wettability.

Suitable cosmetically relevant constituents of the formulation according to the invention can be chosen from the group comprising
a) UV light protection filters,
b) dyestuffs and pigments,
c) moisture-retaining agents/skin-moisturizing agents,
d) deodorant and antiperspirant active compounds,
e) biogenic substances,
f) insect repellent active compounds,
g) hydrotropic agents,
h) antidandruff active compounds,
i) bleaching or skin-lightening agents as well as self-tanning agents,
j) preservatives,
k) surfactants/emulsifiers,
l) perfume oils and plant extracts and/or
m) active compounds.

The content of cosmetically relevant constituents in the formulation according to the invention can be 1-25 wt.%.

The content of water in the formulation according to the invention can preferably be greater than 70 wt.% and particularly preferably greater than 85 wt.%, in each case based on the total amount of the formulation.

### a) UV light protection filters

UV light protection filters, according to the invention, are organic substances (light protection filters) which are liquid or crystalline at room temperature and are capable of absorbing ultraviolet rays and of releasing the absorbed energy again in the form of radiation of longer wavelength, for example heat. UV filters can be oil-soluble or water-soluble. Oil-soluble substances which may be mentioned are, for example:
- 3-benzylidenecamphor and 3-benzylidenenorcamphor and derivatives thereof, for example 3-(4-methylbenzylidene)camphor as described in EP 0 693 471 B1
- 4-aminobenzoic acid derivatives, preferably 4-(dimethylamino)benzoic acid 2-ethylhexyl ester, 4-(dimethylamino)benzoic acid 2-octyl ester and 4-(dimethylamino)benzoic acid amyl ester
- esters of cinnamic acid, preferably 4-methoxycinnamic acid 2-ethylhexyl ester, 4-methoxycinnamic acid propyl ester, 4-methoxycinnamic acid isoamyl ester, 2-cyano-3,3-phenylcinnamic acid 2-ethylhexyl ester (octocrylene)
- esters of salicylic acid, preferably salicylic acid 2-ethylhexyl ester, salicylic acid 4-isopropylbenzyl ester, salicylic acid homomenthyl ester
- derivatives of benzophenone, preferably 2-hydroxy-4-methoxybenzophenone, 2-hydroxy-4-methoxy-4'-methylbenzophenone, 2,2'-dihydroxy-4-methoxybenzophenone
- esters of benzalmalonic acid, preferably 4-methoxybenzalmalonic acid di-2-ethylhexyl ester
- triazine derivatives, such as, for example, 2,4,6-trianilino-(p-carbo-2'-ethyl-1'-hexyloxy)-1,3,5-triazine and octyl triazone, as described in EP 0 818 450 A1, or dioctyl butamido triazone (Uvasorb TM HEB)
- propane-1,3-diones, such as, for example, 1-(4-tert-butylphenyl)-3-(4'-methoxyphenyl)propane-1,3-dione
- ketotricyclo(5.2.1.0)decane derivatives, as described in EP 0 694 521 B1.

Possible water-soluble substances are:
- 2-phenylbenzimidazole-5-sulfonic acid and alkali metal, alkaline earth metal, ammonium, alkylammonium, alkanolammonium and glucammonium salts thereof
- sulfonic acid derivatives of benzophenones, preferably 2-hydroxy-4-methoxybenzophenone-5-sulfonic acid and its salts
- sulfonic acid derivatives of 3-benzylidenecamphor, such as, for example, 4-(2-oxo-3-bornylidenemethyl)benzenesulfonic acid and 2-methyl-5-(2-oxo-3-bornylidene)sulfonic acid and salts thereof.

Possible typical UV-A filters are, in particular, derivatives of benzoylmethane, such as, for example, 1-(4'-tert-butylphenyl)-3-(4'-methoxyphenyl)propane-1,3-dione, 4-tert-butyl-4'-methoxydibenzoylmethane (Parsol TM 1789), 1-phenyl-3-(4'-isopropylphenyl)-propane-1,3-dione as well as enamine compounds, as described in DE 191 12 033 A1 (BASF). The UV-A and UV-B filters can of course also be employed in mixtures. Particularly favourable combinations comprise the derivatives of benzoylmethane, for example 4-tert-butyl-4'-methoxydibenzoylmethane (Parsol TM 1789) and 2-cyano-3,3-phenylcinnamic acid 2-ethyl-hexyl ester (octocrylene) in combination with esters of cinnamic acid, preferably 4-methoxycinnamic acid 2-ethylhexyl ester and/or 4-methoxycinnamic acid propyl ester and/or 4-methoxycinnamic acid isoamyl ester. Such combinations are advantageously combined with water-soluble filters, such as, for example, 2-phenylbenzimidazole-5-sulfonic acid and alkali metal, alkaline earth metal, ammonium, alkylammonium, alkanolammonium and glucammonium salts thereof.

UV filters which can be dissolved in the aqueous phase or emulsified therein are particularly advantageous according to the invention.

In addition to the soluble substances mentioned, insoluble light protection pigments, namely finely disperse metal oxide powders, which are preferably in hydrophobized form, and salts are also possible for this purpose. Examples of suitable metal oxide powders or hydrophobized metal oxide powders can be titanium dioxide powder, aluminium oxide powder, zinc oxide powder and/or a mixed oxide powder with the elements Si, Ti, Al, Zn, Fe, B, Zr and/or Ce.

The content of metal oxide powder, based on the sum of hydrophobized silicon dioxide powder and metal oxide powder, is preferably less than 50 wt.% and particularly preferably less than 30 wt.%.

Silicates (talc), barium sulfate or zinc stearate can furthermore be employed.

So-called micro- or nanopigments are preferably employed in sunscreen compositions. The particles should have an average diameter of less than 100 nm, preferably between 5 and 50 nm and in particular between 15 and 30 nm. They can have a spherical form, but those particles which have an ellipsoidal form or a form which deviates otherwise from the spherical shape can also be employed.

Typical examples are coated titanium dioxides, such as, for example, UV-Titan M212, M 262 and X 111 (Kemira), AEROXIDE TiO2 P25, PF2, T 805 and T 817 (Degussa), Micro Titanium Dioxide MT-150 W, MT-100 AQ, MT-100 SA, MT-100 HD, MT-100 TV (Tayca), Eusolex TM T2000 (Merck), Zinc Oxide neutral H&R and Zinc Oxide NDM (Haarmann & Reimer) as well as Z-Cote and Z-Cote HP1 (BASF). Dispersions, such as, for example, TEGO Sun TAQ 40, a 40 wt.% strength aqueous dispersion of a hydrophobized titanium dioxide (Degussa) can also be used. Further suitable UV light protection filters are to be found in the overview by P. Finkel in SÖFW-Journal 122, 543 (1996) as well as Parf. Kosm. 3,11 (1999). Optical brighteners, such as, for example, 4,4'-diaminostilbene-2,2'-disulfonic acid and its derivatives, can moreover be used.

In addition to the two abovementioned groups of primary light protection substances, secondary light protection agents of the antioxidant type, which interrupt the photochemical reaction chain which is initiated when UV radiation penetrates into the skin, can also be employed. Typical examples of these are amino acids (for example glycine, histidine, tyrosine, tryptophan) and derivatives thereof, imidazoles (for example urocanic acid) and derivatives thereof, peptides, such as D,L-carnosine, D-carnosine, L-carnosine and derivatives thereof (for example anserine), carotenoids, carotenes (for example alpha-carotene, beta-carotene, lycopene) and derivatives thereof, chlorogenic acid and derivatives thereof, liponic acid and derivatives thereof (for example dihydroliponic acid), aurothioglucose, propylthiouracil and other thiols (for example thioredoxin, glutathione, cysteine, cystine, cystamine and glycosyl, N-acetyl, methyl, ethyl, propyl, amyl, butyl and lauryl, palmitoyl, oleyl, gamma-linoleyl, cholesteryl and glyceryl esters thereof) and salts thereof, dilauryl thiodipropionate, distearyl thiodipropionate, thiodipropionic acid and derivatives thereof (esters, ethers, peptides, lipids, nucleotides, nucleosides and salts) as well as sulfoximine compounds (for example buthionine sulfoximine, homocysteine sulfoximine, buthionine sulfones, penta-, hexa-, heptathionine sulfoximine) in very low tolerated dosages (for example pmol to mu mol/kg), furthermore (metal) chelators (for example alpha-hydroxy-fatty acids, palmitic acid, phytic acid, lactoferrin), alpha-hydroxy acids (for example citric acid, lactic acid, malic acid), humic acid, bile acid, bile extracts, bilirubin, biliverdin, EDTA, EGTA and derivatives thereof, unsaturated fatty acids and derivatives thereof (for example gamma-linolenic acid, linoleic acid, oleic acid), folic acid and derivatives thereof, ubiquinone and ubiquinol and derivatives thereof, vitamin C and derivatives (for example ascorbyl palmitate, Mg ascorbyl phosphate, ascorbyl acetate), tocopherols and derivatives (for example vitamin E acetate), vitamin A and derivatives (vitamin A palmitate) as well as coniferylbenzoate of benzoin resin, rutic acid and derivatives thereof, alpha-glycosylrutin, ferulic acid, furfurylideneglucitol, carnosine, butylhydroxytoluene, butylhydroxyanisole, nordihydroguaiac resin acid, nordihydroguaiaretic acid, trihydroxybutyrophenone, uric acid and derivatives thereof, mannose and derivatives thereof, superoxide dismutase, zinc and derivatives thereof (for example ZnO, ZnSO₄), selenium and derivatives thereof (for example selenium methionine), stilbenes and derivatives thereof (for example stilbene oxide, trans-stilbene oxide) and the derivatives suitable according to the invention (salts, esters, ethers, sugars, nucleotides, nucleosides, peptides and lipids) of these active compounds mentioned.

### b) Dyestuffs and pigments

Dyestuffs which can be used according to the invention are natural plant or animal dyestuffs, such as, for example, betanin, bixin, carmine, carotene, chlorophyll, sepia etc. and derivatives thereof as well as synthetic organic dyestuffs, such as, for example, azo, anthraquinone, triphenylmethane dyestuffs etc. Water-soluble or water-dispersible dyestuffs can be particularly preferred.

The formulation according to the invention can also comprise inorganic pigments, such as ochre, umbra, red bolus, terra di siena, chalk etc., as well as synthetic inorganic pigments, such as iron oxides, ultramarine, titanium dioxide, zinc oxide, mica-based pigments, such as, for example, pearlescent pigments. Water-wettable pigments can be particularly preferred.

### c) Moisture-retaining agents/skin-moisturizing agents

In a preferred embodiment, the formulation according to the invention also comprises a moisture-retaining agent. This serves to further optimize the sensory properties of the composition and to regulate the moisture of the skin. At the same time, the low temperature stability of the formulations according to the invention is increased, especially in the case of emulsions. The moisture-retaining agents are conventionally present in an amount of 0.1-15 wt.%, preferably 1-10 wt.%, and in particular 5-10 wt.%.

Agents which are suitable according to the invention are, inter alia, amino acids, pyrrolidonecarboxylic acid, lactic acid and salts thereof, lactitol, urea and urea derivatives, uric acid, glucosamine, creatinine, cleavage products of collagen, chitosan or chitosan salts/ derivatives, and in particular polyols and polyol derivatives (for example glycerol, diglycerol, triglycerol, ethylene glycol, propylene glycol, butylene glycol, pentylene glycol, erythritol, 1,2,6-hexanetriol, polyethylene glycols, such as PEG-4, PEG-6, PEG-7, PEG-8, PEG-9, PEG-10, PEG-12, PEG-14, PEG-16, PEG-18, PEG-20), sugars and sugar derivatives (inter alia fructose, glucose, maltose, maltitol, mannitol, inositol, sorbitol, sorbitylsilanediol, sucrose, trehalose, xylose, xylitol, glucuronic acid and salts thereof), ethoxylated sorbitol (sorbeth-6, sorbeth-20, sorbeth-30, sorbeth-40), honey and hydrogenated honey, hydrogenated starch hydrolysates as well as mixtures of hydrogenated wheat protein and PEG-20 acetate copolymer. The formulation according to the invention can particularly preferably comprise glycerol, diglycerol, triglycerol and butylene glycol.

### d) Deodorant and antiperspirant active compounds

Deodorant and antiperspirant active compounds can also be added according to the invention. These active compounds include astringent metal salts (antiperspirant active compounds), germ-inhibiting agents, enzyme inhibitors, odour absorbers, odour maskers or any desired combination of these active compounds. The formulation according to the invention can comprise the deodorant/antiperspirant active compounds in an amount of 0.1-30 wt.%, preferably 5-25 wt.% and in particular 10-25 wt.% (based on the amount of the formulation).

Possible antiperspirant active compounds are, for example, aluminium hydrochlorides, aluminium zirconium hydrochlorides as well as zinc salts. In addition to the hydrochlorides, the formulation according to the invention can also comprise aluminium hydroxylactates as well as acid aluminium/zirconium salts, for example Locron TM (formula [Al₂(OH)₅Cl] x 2.5 H₂O, Clariant GmbH) or Rezal TM 36G (aluminium zirconium tetrachlorohydrex-glycine complex, Reheis).

Enzyme inhibitors, for example esterase inhibitors, can be added as further deodorant active compounds. These are preferably trialkyl citrates, such as trimethyl citrate, tripropyl citrate, triisopropyl citrate, tributyl citrate and, in particular, triethyl citrate (Hydagen TM C. A. T., Cognis Deutschland GmbH). The substances inhibit the enzyme activity of bacteria which decompose perspiration, and thereby reduce odour formation. Further substances which are possible esterase inhibitors are sterol sulfates or phosphates, such as, for example, lanosterol, cholesterol, campesterol, stigmasterol and sitosterol sulfate and phosphate, dicarboxylic acids and esters thereof, such as, for example, glutaric acid, glutaric acid monoethyl ester, glutaric acid diethyl ester, adipic acid, adipic acid monoethyl ester, adipic acid diethyl ester, malonic acid and malonic acid diethyl ester, hydroxycarboxylic acids and esters thereof, such as, for example, citric acid, malic acid, tartaric acid or tartaric acid diethyl ester. The formulation according to the invention can likewise comprise antibacterial active compounds which influence the germ flora and kill or inhibit the growth of bacteria which decompose perspiration. Examples of these are chitosan, phenoxyethanol, chlorhexidine gluconate or 5-chloro-2-(2,4-dichlorophenoxy)-phenol (Irgasan TM, Ciba-Geigy, Basle/CH).

Suitable germ-inhibiting agents are in principle all the substances which are active against Gram-positive bacteria, such as, for example, 4-hydroxybenzoic acid and its salts and esters, N-(4-chlorophenyl)-N'-(3,4-dichlorophenyl)urea, 2,4,4'-trichloro-2'-hydroxy-diphenyl ether (Triclosan), 4-chloro-3,5-dimethyl-phenol, 2,2'-methylene-bis(6-bromo-4-chlorophenol), 3-methyl-4-(1-methylethyl)-phenol, 2-benzyl-4-chlorophenol, 3-(4-chlorophenoxy)-1,2-propanediol, 3-iodo-2-propynyl butylcarbamate, chlorhexidine, 3,4,4'-trichlorocarbanilide (TTC), antibacterial odoriferous substances, thymol, thyme oil, eugenol, clove oil, menthol, mint oil, farnesol, phenoxyethanol, glycerol monocaproate, glycerol monocaprylate, glycerol monolaurate (GML), diglycerol monocaproate (DMC), salicylic acid N-alkylamides, such as, for example, salicylic acid n-octylamide or salicylic acid n-decylamide.

Suitable odour absorbers are substances which can absorb and largely retain odour-forming compounds. They lower the partial pressure of the individual components and in this way also reduce the rate at which they spread. It is important that perfumes must remain out of consideration in this context. They comprise, for example, as the main constituent a complex zinc salt of ricinoleic acid or specific fragrances of largely neutral odour which are known to the person skilled in the art as "fixatives", such as, for example, extracts of labdanum or styrax or certain abietic acid derivatives.

Odoriferous substances or perfume oils which function as odour maskers are those which, in addition to their function as odour maskers, impart to the deodorants their particular fragrance note. Examples of perfume oils which may be mentioned are mixtures of natural and synthetic odoriferous substances. Natural odoriferous substances are extracts of flowers, stems and leaves, fruits, pericarp, roots, woody plants, herbs and grasses, needles and branches as well as resins and balsams. Animal raw materials are furthermore possible, such as, for example, civet and castoreum. Typical synthetic odoriferous compounds are products of the ester, ether, aldehyde, ketone, alcohol and hydrocarbon type. Odoriferous compounds of the ester type are, for example, benzyl acetate, p-tert-butylcyclohexyl acetate, linalyl acetate, phenylethyl acetate, linalyl benzoate, benzyl formate, allylcyclohexyl propionate, styrallyl propionate and benzyl salicylate. The ethers include, for example, benzyl ethyl ether, the aldehydes include, for example, the linear alkanals having 8 to 18 carbon atoms, citral, citronellal, citronellyloxyacetaldehyde, cyclamenaldehyde, hydroxycitronellal, lilial and bourgeonal, the ketones include, for example, the jonones and methyl cedryl ketone, the alcohols include anethole, citronellol, eugenol, isoeugenol, geraniol, linalool, phenylethyl alcohol and terpineol, the hydrocarbons include chiefly the terpenes and balsams. Preferably, however, mixtures of various odoriferous substances which together generate a pleasing fragrance note are used. Essential oils of relatively low volatility, which are usually used as aroma components, are also suitable as perfume oils, for example sage oil, camomile oil, clove oil, melissa oil, mint oil, cinnamon leaf oil, linden blossom oil, juniper berry oil, vetiver oil, oliban oil, galbanum oil, labdanum oil and lavandine oil. Bergamot oil, dihydromyrcenol, lilial, lyral, citronellol, phenylethyl alcohol, alpha-hexylcinnamaldehyde, geraniol, benzylacetone, cyclamenaldehyde, linalool, Boisambrene Forte, ambroxan, indole, hedione, sandelice, lemon oil, mandarin oil, orange oil, allylamyl glycollate, cyclovertal, lavandine oil, muskat grape, sage oil, beta-damascone, geranium oil, bourbon, cyclohexyl salicylate, Vertofix coeur, Iso-E-super, Fixolide NP, evernyl, iraldein gamma, phenylacetic acid, geranyl acetate, benzyl acetate, rose oxide, romilat, irotyl and floramat, by themselves or in mixtures, are preferably employed.

### e) Biogenic substances

Biogenic active compounds which are suitable according to the invention are, for example, tocopherol, tocopherol acetate, tocopherol palmitate, ascorbic acid, (deoxy)ribonucleic acid and fragmentation products thereof, beta-glucans, retinol, bisabolol, allantoin, phytantriol, panthenol, pantothenic acid, fruit acids, alpha-hydroxy acids, amino acids, ceramides, pseudoceramides, essential oils, plant extracts, such as, for example, prunus extract, bambara nut extract and vitamin complexes.

### f) Insect repellent active compounds

A further preferred embodiment of the formulation according to the invention additionally comprises at least one insect repellent active compound or a combination of these active compounds. Possible insect repellents are, for example, N,N-diethyl-m-toluamide, 1,2-pentanediol or 3-(N-n-butyl-N-acetyl-amino)-propionic acid ethyl ester) (Insect Repellent 3535, Merck KGaA), as well as butyl acetylaminopropionate. They are conventionally present in the formulation according to the invention in an amount of 0.1-10 wt.%, preferably 1-8 wt.%, and particularly preferably in an amount of 2-6 wt.%, based on the formulation.

### g) Hydrotropic agents

Hydrotropic agents, such as, for example, ethanol, isopropyl alcohol, or polyols, can furthermore be present according to the invention. Polyols which are possible here preferably have 2 to 15 carbon atoms and at least two hydroxyl groups. The polyols can also contain further functional groups, in particular amino groups, or can be modified with nitrogen. Typical examples are:
- glycerol
- alkylene glycols, such as, for example, ethylene glycol, diethylene glycol, propylene glycol, butylene glycol, pentylene glycol, hexylene glycol as well as polyethylene glycols having an average molecular weight of from 100 to 1,000 Daltons.
- technical-grade oligoglycerol mixtures having an intrinsic degree of condensation of from 1.5 to 10, such as, for example, technical-grade diglycerol mixtures having a diglycerol content of from 40 to 50 wt.%.
- methylol compounds, such as, in particular, trimethylolethane, trimethylolpropane, trimethylolbutane, pentaerythritol and dipentaerythritol
- short-chain alkyl glucosides, in particular those having 1 to 8 carbon atoms in the alkyl radical, such as, for example, methyl and butyl glucoside
- sugar alcohols having 5 to 12 carbon atoms, such as, for example, sorbitol or mannitol
- sugars having 5 to 12 carbon atoms, such as, for example, glucose or sucrose
- amino sugars, such as, for example, glucamine
- dialcoholamines, such as diethanolamine or 2-amino-1,3-propanediol.

### h) Antidandruff active compounds

Possible antidandruff active compounds in the formulation according to the invention are piroctone olamine (1-hydroxy-4-methyl-6-(2,4,4-trimethylpentyl)-2-(1H)-pyridinone monoethanolamine salt), Baypival TM (climbazole), Ketoconazol TM, (4-acetyl-1- -4-[2-(2,4-dichlorophenyl) r-2-(1H-imidazol-1-ylmethyl)-1,3-dioxylan-c-4-ylmethoxyphenylpiperazine, ketoconazole, Elubiol, selenium disulfide, colloidal sulfur, sulfur-polyethylene glycol sorbitan monooleate, sulfur-castor oil polyethoxylate, sulfur tar distillates, salicylic acid (and in combination with hexachlorophene), undecylenic acid monoethanolamide sulfosuccinate Na salt, Lamepon TM UD (protein-undecylenic acid condensate), zinc pyrithione, aluminium pyrithione and magnesium pyrithione/dipyrithione magnesium sulfate.

### i) Bleaching or skin-lightening agents as well as self-tanning agents

The formulation according to the invention can comprise bleaching or skin-lightening agents, such as, for example, basic bismuth salts, hydroquinone, compounds which split off oxygen, such as, for example, zinc peroxide, urea peroxide, hydrogen peroxide and/or organic peroxides. The formulation according to the invention can particularly preferably comprise hydrogen peroxide, which is employed in the form of aqueous solutions. Possible tyrosinase inhibitors, which prevent the formation of melanin and are used in depigmentation agents, are, for example, arbutin, ferulic acid, koji acid, coumaric acid and ascorbic acid (vitamin C, sodium ascorbyl phosphate, magnesium ascorbyl phosphate). Cosmocair C 250 from Degussa AG is particularly suitable.
Dihydroxyacetone, for example, is suitable as a self-tanning agent.

### j) Preservatives

Suitable preservatives are, for example, phenoxyethanol, formaldehyde solution, parabens, pentanediol or sorbic acid and the silver complexes known under the name Surfacine TM and further substance classes listed in Appendix 6, Part A and B of the cosmetics legislation.

### k) Surfactants/emulsifiers

The formulation according to the invention can comprise surfactants/emulsifiers. However, the amount of these substances in the formulation is critical, since their wetting properties may prevent the formation of a powder on addition of hydrophobized silicon dioxide powder, and therefore no formulation according to the invention can be obtained. As a rule, the formulations according to the invention therefore contain no surfactants/emulsifiers. If the formulation according to the invention is to comprise these, the amount must be optimized according to the formulation.

The nature of the surfactants/emulsifiers is not limited. Thus, a formulation according to the invention can comprise nonionic, zwitter-ionic, amphoteric, cationic and furthermore anionic surfactants.

### l) Perfume oils and plant extracts

The formulation according to the invention can comprise perfume oils. These can be natural, plant and animal as well as synthetic odoriferous substances or mixtures thereof. Natural odoriferous substances are obtained, inter alia, by extraction of flowers, stems, leaves, fruits, pericarp, roots and resins of plants. Animal raw materials are furthermore possible, such as, for example, civet and castoreum. Typical synthetic odoriferous compounds are products of the ester, ether, aldehyde, ketone, alcohol and hydrocarbon type. Mixtures of various odoriferous substances which together generate a pleasing fragrance note are preferably used.

Plant extracts which can be employed according to the invention include, for example, extracts of arnica, birch, camomile, burdock root, beard lichen, poplar, stinging nettle and of walnut shells.

### m) Active compounds

The formulation according to the invention can comprise hormones, such as, for example, oxytocin, corticotropin, vasopressin, secretin, gastrin.

The formulation according to the invention can furthermore comprise hydrophobic, organic powders of polystyrenes, polyethylenes, organopolysiloxanes, polymethylsilsesquioxanes, N-acyllysine, polyethylene tetrafluoride resins, acrylic acid resins, epoxy resins, polymethyl methacrylates, acrylonitrile/methacrylate copolymers, vinylidene chloride/methacrylic acid copolymers and/or nylon powder.

The formulation according to the invention can preferably comprise hydrophobized inorganic metal oxide powders, such as, for example, titanium dioxide or aluminium oxide, and it may be particularly preferable if these metal oxide powders are of pyrogenic origin. The content of these hydrophobized metal oxide powders is preferably less than 50 wt.% and particularly preferably less than 30 wt.%, in each case based on the sum of hydrophobized silicon dioxide powder and hydrophobized metal oxide powder.

The formulation according to the invention can furthermore comprise viscosity regulators. These can preferably be:
hydrogel-forming agents or hydrocolloids, such as, for example, modified polysaccachrides, such as cellulose ethers and cellulose esters, for example carboxymethylcellulose, methylcellulose, hydroxyethylcellulose, methylhydroxyethylcellulose, methylhydroxypropylcellulose, xanthan gum, guar-guar, agar-agar, alginates and tyloses;
inorganic hydrocolloids, such as bentonites, magnesium aluminium silicates, silicon dioxide; as well as synthetic hydrocolloids, such as polyacrylates (for example Carbopols TM and Pemulen types from Goodrich; Synthalens TM from Sigma; Keltrol types from Kelco; Sepigel types from Seppic; Salcare types from Allied Colloids), non-crosslinked and polyol-crosslinked polyacrylic acids, polyacrylamides, polyvinyl alcohol and polyvinylpyrrolidone.

Surfactants, such as, for example, ethoxylated fatty acid glycerides, esters of fatty acids with polyols, such as, for example, pentaerythritol or trimethylolpropane, fatty alcohol ethoxylates having a narrowed distribution of homologues, alkyl oligoglucosides as well as electrolytes, such as, for example, sodium chloride and ammonium chloride, can also be employed for regulating the viscosity.

Anionic zwitter-ionic, amphoteric and nonionic copolymers, such as, for example, vinyl acetate/crotonic acid copolymers, vinylpyrrolidone/vinyl acrylate copolymers, vinyl acetate/butyl maleate/isobornyl acrylate copolymers, methyl vinyl ether/maleic anhydride copolymers and esters thereof, acrylamidopropyltrimethylammonium chloride/ acrylate copolymers, octylacrylamide/methyl methacrylate/tert-butylaminoethyl methacrylate/2-hydroxypropyl methacrylate copolymers, vinylpyrrolidone/ vinyl acetate copolymers, vinylpyrrolidone/ dimethylaminoethyl methacrylate/vinylcaprolactam terpolymers as well as optionally derivatized cellulose ethers and silicones are also suitable as viscosity regulators. Further suitable polymers and thickening agents are listed in Cosm. Toil. 108, 95 (1993).

The content of the viscosity regulator in the formulation according to the invention can be up to 20 wt.%, preferably 1 - 5 wt.%.

The formulation according to the invention can furthermore comprise at least one oily substance. According to the invention, oily substances are to be understood as meaning substances or mixtures of substances which are liquid at 20 ºC and are immiscible with water at 25 ºC. Combination with oily substances allows optimization of the sensory properties of the formulations.

Possible oily substances are, for example, Guerbet alcohols based on fatty alcohols having 6 to 18, preferably 8 to 10 carbon atoms (for example Eutanol TM G), esters of linear C₆-C₂₂-fatty acids with linear or branched C₆-C₂₂-fatty alcohols and esters of branched C₆-C₁₃-carboxylic acids with linear or branched C₆-C₂₂-fatty alcohols, such as, for example, myristyl myristate, myristyl palmitate, myristyl stearate, myristyl isostearate, myristyl oleate, myristyl behenate, myristyl erucate, cetyl myristate, cetyl palmitate, cetyl stearate, cetyl isostearate, cetyl oleate, cetyl behenate, cetyl erucate, stearyl myristate, stearyl palmitate, stearyl stearate, stearyl isostearate, stearyl oleate, stearyl behenate, stearyl erucate, isostearyl myristate, isostearyl palmitate, isostearyl stearate, isostearyl isostearate, isostearyl oleate, isostearyl behenate, isostearyl oleate, oleyl myristate, oleyl palmitate, oleyl stearate, oleyl isostearate, oleyl oleate, oleyl behenate, oleyl erucate, behenyl myristate, behenyl palmitate, behenyl stearate, behenyl isostearate, behenyl oleate, behenyl behenate, behenyl erucate, erucyl myristate, erucyl palmitate, erucyl stearate, erucyl isostearate, erucyl oleate, erucyl behenate and erucyl erucate. In addition, esters of linear C₆-C₂₂-fatty acids with branched alcohols, in particular 2-ethylhexanol, esters of C₃-C₃₈-alkylhydroxycarboxylic acids with linear or branched C₆-C₂₂-fatty alcohols - in particular diethylhexyl malate - , esters of linear and/or branched fatty acids with polyhydric alcohols (such as, for example, propylene glycol, dimer diol or trimer triol) and/or Guerbet alcohols, triglycerides based on C₆-C₁₀-fatty acids, liquid mono-/di-/triglyceride mixtures based on C₆-C₁₈-fatty acids, esters of C₆-C₂₂-fatty alcohols and/or Guerbet alcohols with aromatic carboxylic acids, in particular benzoic acid, esters of C₂-C₁₂-dicarboxylic acids with linear and/or branched alcohols having 1 to 22 carbon atoms or polyols having 2 to 10 carbon atoms and 2 to 6 hydroxyl groups, plant oils, branched primary alcohols, substituted cyclohexanes, linear and branched C₆-C₂₂-fatty alcohol carbonates, such as, for example, dicaprylyl carbonate (Cetiol TM CC), Guerbet carbonates based on fatty alcohols having 6 to 18, preferably 8 to 10 C atoms, esters of benzoic acid with linear and/or branched C₆-C₂₂-alcohols (for example Finsolv TM TN), linear or branched, symmetric or unsymmetric dialkyl ethers having 6 to 22 carbon atoms per alkyl group, such as, for example, dicaprylyl ether (Cetiol TM OE), ring-opening products of epoxidized fatty acid esters with polyols (Hydagen TM HSP, Sovermol TM 750, Sovermol TM 1102), and/or aliphatic or naphthenic hydrocarbons, such as, for example, mineral oil, Vaseline, petrolatum, squalane, squalene, dialkyl ethers, dialkyl carbonates and/or dialkylcyclohexanes, are suitable.

The formulation according to the invention can moreover comprise silicone compounds. These can be cyclomethicone, dimethicone, dimethylpolysiloxanes, methylphenylpolysiloxanes, cyclic silicones, silicone compounds modified by amino, fatty acid, alcohol, polyether, epoxy, fluorine, glycoside and/or alkyl. Simethicones, which are mixtures of dimethicones having an average chain length of from 200 to 300 dimethylsiloxane units and silicon dioxide or hydrogenated silicates, are furthermore suitable.

The amount of oily substances in the total composition can be between 0.1 and 10 wt.%, depending on the application form. The amount can particularly preferably vary between 0.5 and 3 wt.%.

### Examples

88.92 g deionized water and 5.0 g propylene glycol (Caelo) are initially introduced into a glass beaker, 0.8 g LCW Covagel (LCW Sensient) is added, while stirring with a magnetic stirrer, and this mixture is then stirred at room temperature for a further 15 minutes. The clear solution is introduced into an appropriate 500 ml high-grade steel beaker, 5.0 g of the hydrophobized silicon dioxide powder, optionally together with a further metal oxide powder, are added (see Table 2) and the components are mixed with a dissolver (DISPERMAT®; VMA-Getzmann, disc diameter 5 cm) for 60 seconds at 10,000 revolutions per minute. The pulverulent product obtained is then bottled in 250 ml screw-cap bottles and stored in the tightly closed vessels for three months at 0 ºC, 23 ºC and 40 ºC. The consistency of the product is evaluated visually.

The hydrophobized silicon dioxide powders employed are shown in Table 1.
It can be seen from Table 2 that the formulations according to the invention of Examples 3 to 8 have outstanding storage stabilities both at 0 ºC, 23 ºC and 40 ºC. Only in the formulations of Examples 5 and 8 are a few drops of liquid found on the lid, base and/or container walls on storage at 40 ºC.
In contrast, the formulations of Comparison Examples 1 and 2, in which the same hydrophobized silicon dioxide powder as in Examples 3 and 4 according to the invention is employed, but with a lower tamped density, show a significantly poorer storage stability.
Working with the hydrophobized silicon dioxide powders having a tamped density of at least 70 g/l furthermore leads to only a low formation of dust, and the high flowability of these powders allows a higher metering accuracy during preparation of the formulations.

According to the present invention, it is furthermore also possible for the formulations also to be able to comprise other hydrophobized metal oxide powders, in addition to hydrophobized silicon dioxide powder. In this case also, a high storage stability is ensured.

**Table 1: Substances employed**

| **Hydrophobized SiO₂** | **Hydrophobizing** **agent** | **Spec.** **surface** **area** **(approx.)** **m²/g** | **Methanol wettability** |
|---|---|---|---|
| Aerosil^{®} * | octamethyl-cyclotetrasiloxane | 150 | 40 |
| R104 | | | |
| Aerosil^{®} * | octamethyl-cyclotetrasiloxane | 250 | 45 |
| R106 | | | |
| Aerosil^{®} * | polydimethyl-siloxane | 100 | 70 |
| R202 | | | |
| Aerosil^{®} * | octylsilane | 150 | 45 |
| R805 | | | |
| Aerosil^{®} | hexamethyl-disilazane | 260 | 50 |
| R812 | | | |
| Aerosil^{®} | hexamethyl-disilazane | 220 | 60 |
| R812S | | | |
| Aerosil^{®} | hexamethyl-disilazane | 150 | 65 |
| R8200 | | | |

| | | | |
|---|---|---|---|
| *) reference SiO₂, excluded from present invention. | | | |

**Table 2: Composition and properties of the formulations**

| | | **Comparison According to the invention** | | | | | | | |
|---|---|---|---|---|---|---|---|---|---|
| **Example** | | **1** | **2** | **3** | **4** | **5** | **6** | **7** | **8** |
| AEROSIL® R | | 812 S | 812 S | 812 S | 812 S | 8200 | 812 S | 812 S | 812 S |
| | | | VV 60 | VV 75 | W 90 | | VV 90 | VV 90 | VV 90 |
| Tamped density [g/l] | | 50-60 | 60 | 75 | 90 | 140 | 90 | 90 | 90 |
| BET surface area [m²/g] | | 220 | 220 | 220 | 220 | 150 | 220 | 220 | 220 |
| Methanol wettability | | 60 | 60 | 60 | 60 | 65 | 60 | 60 | 60 |
| AEROXIDE®* | | - | - | - | - | - | TiO2 T | Alu C^{§} | - |
| | | | | | | | 805 | | |
| Tamped density [g/l] | | - | - | - | - | - | 200 | 80 | - |
| AEROPERL®* R | | - | - | - | - | - | - | - | 806/30^{&} |
| Tamped density [g/l] | | - | - | - | - | - | - | - | 300 |

| Storage stability after 3 months^{(x)} | | | | | | | | | |
|---|---|---|---|---|---|---|---|---|---|
| | 0 ºC | C | C | A | A | A | A | A | A |
| | 23 ºC | A | A | A | A | A | A | A | A |
| | 40 ºC | D | C | B | A | B | A | A | B |

| | | | | | | | | | |
|---|---|---|---|---|---|---|---|---|---|
| *) 8 parts by weight of hydrophobized silicon dioxide powder + 2 parts by weight of AEROXIDE or AEROPERL; § Hydrophobized with hexamethyldisilazane; & 806/30 AEROPERL 300/30 hydrophobized with hexamethyldisilazane x) Evaluation of the storage stability: A = unchanged; B = occurrence of a few drops of liquid on the lid, base and/or the container walls; C = slight phase separation; D = considerable phase separation | | | | | | | | | |

## Claims

1. Pulverulent cosmetic formulation comprising at least one hydrophobized silicon dioxide powder, at least one cosmetically relevant constituent and more than 85 wt.% of water, based on the total amount of the formulation, **characterized in that** the hydrophobized silicon dioxide powder
is a silanized silicon dioxide powder of pyrogenic origin obtained by using hexamethyl disilazane as silanizing agent having
a tamped density of 70 g/l to 250 g/l, determined in accordance with DIN EN ISO 787-11 a methanol wettability of at least 40 and
a BET surface area of from 100 to 400 m²/g.

2. Pulverulent cosmetic formulation according to claim 1, **characterized in that** it comprises up to 20 wt.% of a viscosity regulator.

3. Pulverulent cosmetic formulation according to claims 1 or 2, **characterized in that** it comprises up to 20 wt.% of an oily substance.

## Patentansprüche

1. Pulverförmige kosmetische Zubereitung, enthaltend mindestens ein hydrophobiertes Siliciumdioxidpulver, mindestens einen kosmetisch relevanten Inhaltsstoff und mehr als 85 Gew.-% Wasser, bezogen auf die Gesamtmenge der Zubereitung, **dadurch gekennzeichnet, daß** es sich bei dem hydrophobierten Siliciumdioxidpulver um ein silanisiertes Siliciumdioxidpulver pyrogener Herkunft, das unter Verwendung von Hexamethyldisilazan als Silanisierungsmittel erhalten worden ist, mit einer Stampfdichte von 70 g/l bis 250 g/l, bestimmt nach DIN EN ISO 787-11, einer Methanolbenetzbarkeit von wenigstens 40 und einer BET-Oberfläche von 100 bis 400 m²/g handelt.

2. Pulverförmige kosmetische Zubereitung nach Anspruch 1, **dadurch gekennzeichnet, daß** sie bis zu 20 Gew.-% eines Viskositätsregulators enthält.

3. Pulverförmige kosmetische Zubereitung nach Anspruch 1 oder 2, **dadurch gekennzeichnet, daß** sie bis zu 20 Gew.-% eines Ölkörpers enthält.

## Revendications

1. Composition cosmétique pulvérulente comprenant au moins une poudre de dioxyde de silicium rendue hydrophobe, au moins un constituant cosmétiquement approprié et plus de 85 % en poids d'eau, par rapport à la quantité totale de la composition, **caractérisée en ce que** la poudre de dioxyde de silicium rendue hydrophobe
est une poudre de dioxyde de silicium silanée d'origine pyrogénée, obtenue à l'aide d'hexaméthyldisilazane en tant qu'agent de silanation, ayant
une densité apparente à l'état tassé de 70 g/l à 250 g/l, déterminée selon DIN EN ISO 787-11,
une mouillabilité par le méthanol d'au moins 40 et une surface BET de 100 à 400 m²/g.

2. Composition cosmétique pulvérulente selon la revendication 1, **caractérisée en ce qu'**elle comprend jusqu'à 20 % en poids d'un régulateur de viscosité.

3. Composition cosmétique pulvérulente selon la revendication 1 ou 2, **caractérisée en ce qu'**elle comprend jusqu'à 20 % en poids d'une substance huileuse.
